# FASCICULE DE BREVET EUROPEEN

(11) **EP 1 729 619 B1**
(45) Date de publication et mention de la délivrance du brevet: **12.10.2011**
(21) Numéro de dépôt: 04786286.7
(22) Date de dépôt: 09.08.2004
(51) Int. Cl.: A47K 7/02

(54) **TAMPON POUR LES SOINS DE LA PEAU**
HAUTPFLEGEKISSEN
SKIN CARE PAD

(30) Priorité: 12.08.2003 FR 0309868
(43) Date de publication de la demande: 13.12.2006
(73) Titulaire: Georgia-Pacific France, 92270 Bois-Colombes (FR)
(72) Inventeur: GREGOIRE, Philippe, F-27700 Les Andelys (FR); LOUIS DIT PICARD, Bernard, F-27370 Amfreville la Campagne (FR)
(74) Mandataire: Cortier, Sophie
(86) Numéro de dépôt international: PCT/FR2004/002113
(87) Numéro de publication internationale: WO 2005/016099

(56) Documents cités:
- EP-A- 1 283 019
- EP-A- 1 350 456
- WO-A-01/66345
- WO-A-02/43536
- FR-A- 2 819 390

## Description

### TAMPON POUR LES SOINS DE LA PEAU

La présente invention porte sur un tampon constitué de fibres de coton ou autres matériaux cellulosiques ou synthétiques, destiné aux soins de la peau et ayant en particulier la propriété d'être exfoliant.

Dans le cadre des soins de la peau dans le domaine des produits cosmétiques notamment, on connaît le traitement d'exfoliation ou de gommage. Il s'agit d'une action qui permet l'élimination des impuretés incrustées et accumulées à la surface de l'épiderme, et des cellules mortes par une action mécanique de frottement. Le frottement permet d'affiner le grain de la peau, de purifier l'épiderme et d'éclaircir le teint. On utilise conventionnellement des fibres naturelles. On connaît par exemple le pain de luffa ou courge fibreuse, le gant de crin, de chanvre ou même de sisal. Cependant ces fibres très grossières ont pour effet un gommage fort qui ne peut être réalisé que de temps en temps, de façon hebdomadaire ou encore mensuelle. On connaît aussi des préparations exfoliantes avec des éléments naturels, organiques ou de type végétal comme les akènes de fraise, noyaux d'abricot concassés, silice organique de bambou, cellulose de courge, ou bien encore des éléments de type minéral comme des billes de silice ou bien encore des éléments artificiels et / ou synthétiques. Cela comprend tous les polymères tels que : polyéthylène, nylon, polypropylène, EVA... La tendance actuelle du marché repose sur ces derniers éléments sous forme de microbilles qui permettent des gommages plus doux selon la nature du matériau. On utilise ainsi des sphères cassables larguant des principes actifs.

On connaît le document EP 1 283 019 A1 qui décrit un tampon pour le soin de la peau comprenant des éléments gommants déposés en couche sur l'une de ses surfaces.

Par ailleurs, en tant qu'arrière-plan technologique :

On connaît aussi le document FR 2 819 390 qui divulgue un gant formé en un non-tissé dont l'une des faces est plus rugueuse que l'autre.

Le document WO 01/66345 A1 a pour objet un non tissé formé de trois couches de type airlaid associés thermiquement puis calendrées et gaufrées afin de former des lingettes humides.

La demande de brevet WO 02/43536 montre une lingette non tissée dont chacune des faces présente une texture différente, l'une relativement douce et l'autre relativement abrasive afin de réaliser deux fonctions différentes lors d'opérations de nettoyage ménager.

La présente invention vise un article pour le soin de la peau qui est destiné à un usage quotidien.

Un autre objectif de l'invention est la réalisation d'un article qui peut être utilisé à la fois pour le gommage, le massage et le démaquillage de la peau.

On parvient à cet objectif conformément à l'invention avec un tampon pour le soin de la peau comprenant des éléments gommants répartis entre au moins une première couche fibreuse et une seconde couche fibreuse de plus faible grammage que la première couche fibreuse.

La solution de l'invention permet ainsi d'obtenir un effet gommant du fait de l'application de la surface comportant les éléments gommants sous une couche fibreuse de faible grammage sur la peau avec une dynamique de déplacement.

On obtient un effet massant par la géométrie de la surface et son déplacement sur la peau.

En raison de la couche de fibres de plus faible grammage, l'effet mécanique est atténué ce qui permet d'obtenir un gommage très doux et donc un usage quotidien.

Lorsqu'on emploie des fibres hydrophiles dans au moins l'une des couches, on permet une action renforcée du démaquillage du fait de l'action mécanique des éléments gommants complétée par l'absorption des fibres qui capturent le maquillage. Comme le démaquillage est plus efficace, il est plus rapide. La peau est moins irritée ; elle reste plus souple.

De préférence, les éléments gommants forment une couche, et le grammage de la couche d'éléments gommants est compris entre 2 et 50 g/m². Les éléments gommants sont choisis parmi les produits organiques naturels tels que : akènes de fraise, noyaux d'abricot concassés, silice organique de bambou, cellulose de courge ou encore de façon plus innovante anas de lin, minéraux naturels tels que les billes de silice ou artificiels tels que des sphères de cellulose et de méthylcellulose ou bien encore synthétiques tels que des polymères de polyéthylène, de nylon, de polypropylène ou d'EVA.

Les couches fibreuses sont composées avantageusement de fibres choisies parmi les fibres cellulosiques, artificielles, synthétiques, seules ou en mélange.

En particulier la première couche est composée de fibres choisies parmi les fibres de coton, viscose, polyester, polypropylène, seules ou en mélange, et son grammage est compris entre 20 et 350 g/m². Avantageusement la première couche est une nappe de fibres de type nappage pneumatique, par exemple formée sur une machine du type RANDO WEBBER. Elle peut aussi être composée d'une nappe et d'un voile de carde ou encore de plusieurs voiles de carde.

En particulier également, la seconde couche est composée de fibres choisies parmi les fibres de coton, lin, ramie viscose, polyester, polypropylène, seules ou en mélange, et son grammage est compris entre 5 et 50 g/m². Il est inférieur à celui de la première couche.

Avantageusement, la seconde couche est composée d'un ou plusieurs voiles de carde.

Selon un mode de réalisation particulier, le tampon comprend une deuxième couche d'éléments gommants compris entre la première couche et une troisième couche fibreuse de plus faible grammage que celui de la première couche.

Avantageusement, les couches fibreuses sont liées et en particulier les couches sont liées entre elles par exemple par liage hydraulique ou mécanique ou bien, lorsque les couches comprennent au moins une partie de matière thermofusible, les fibres sont au moins partiellement thermoliées.

L'invention porte également sur un procédé de fabrication d'un tel tampon. Il comprend les étapes suivantes : formation d'une première couche de fibres, dépôt d'une couche d'éléments gommants sur la première couche, dépôt d'une deuxième couche de fibres sur la couche d'éléments gommants la deuxième couche de fibres étant de plus faible grammage que la première couche de fibres.

L'invention sera mieux comprise avec la description qui suit de divers modes de réalisation, en relation avec les dessins annexés sur lesquels
- la figure 1 représente schématiquement la structure d'un tampon de l'invention, vu en coupe transversale et en perspective,
- les figures 2 à 4 représentent des variantes de structure de tampon.

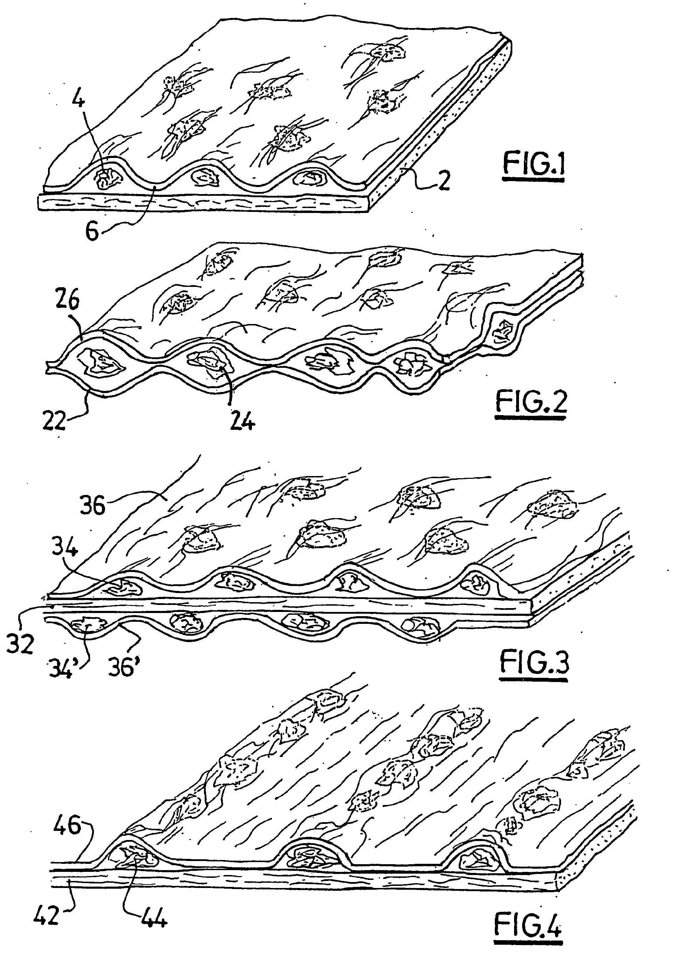

Comme on le voit sur la figure 1, le produit de l'invention est composé d'une première couche de fibres 2 servant de support à une couche d'éléments gommants 4, répartis uniformément ou selon un motif déterminé sur sa surface. Les éléments gommants sont immobilisés par une seconde couche de fibres 6 qui reste suffisamment fine pour ne pas masquer l'effet sur la peau de la couche d'éléments gommants.

Pour la première couche, on emploie de manière préférée une couche de fibres formée par une machine de type Rando Webber qui dispose les fibres avec une certaine orientation inclinée par rapport à la direction de formation de la nappe. La couche est ainsi relativement épaisse. On emploie avantageusement des fibres de coton hydrophile. Cependant comme cela l'a été mentionné plus haut, d'autres catégories de fibres sont possibles. Le grammage de la couche 2 est compris entre 20 et 350g/m².

La couche gommante est composée d'éléments qui sont connus dans le domaine cosmétique comme présentant des propriétés exfoliantes tolérées par la peau. Il peut s'agir comme cela a été mentionné plus haut d'éléments de nature organique, minérale, artificielle ou bien synthétique. Les éléments se présentent sous la forme de fibres, de granulés ou bien de micro-billes ; le grammage de la couche d'éléments gommants 4 est compris entre 2 et 50 g/m².

La couche supérieure 6 est formée d'une deuxième couche fibreuse destinée à prendre en sandwich avec la première couche 2, les éléments de la couche gommante 4. Cette couche est avantageusement constituée d'un ou plusieurs voiles de carde. L'avantage du voile de carde est d'offrir une certaine résistance avant même toute liaison des fibres d'une autre manière, tout en restant assez fin pour ne pas masquer la rugosité des éléments sous-jacents. Le grammage de la couche est compris entre 5 et 50 g/m². Il est de préférence toujours inférieur à celui de la première couche qui sert plutôt de support.

En utilisation, on frotte la peau en appliquant le côté du tampon avec la deuxième couche 6 pour obtenir un effet gommant et massant à la fois. On utilise la seconde face du coté de la première couche de fibres 2 pour l'essuyage par exemple. Elle est nécessairement plus douce, car on ne sent pas ou on les sent moins, les particules gommantes à travers elle.

L'ensemble peut être laissé sous la forme de couches non liées ; mais l'ensemble manquant de cohésion surtout vis à vis de l'action de frottage, on lie de préférence les couches entre elles. Par exemple on peut les lier hydrauliquement ou mécaniquement ou bien par un autre moyen équivalent quant au résultat. Le tampon peut aussi comprendre de la matière thermofusible et les fibres être au moins partiellement thermoliées.

Cependant l'invention ne se limite pas à cette structure. Sur la figure 2, on a représenté un mode de réalisation où la première couche 22 servant de support est aussi un voile de carde ou une pluralité de voiles de carde. On retrouve sur le tampon de la figure 2, la couche 24 d'éléments gommants comme dans le mode de réalisation de la figure 1, et une couche fibreuse supérieure 26.

Selon la réalisation de la figure 3, on a créé un tampon à deux faces exfoliantes, la cohésion de l'ensemble étant assurée par la couche centrale qui est de préférence plus épaisse. Le tampon comprend donc une couche centrale 32 qui peut être un voile de carde mais de préférence, il s'agit d'une nappe comme pour la première couche du mode de réalisation de la figure 1. On trouve une couche 36 et 36' de chaque côté de la première couche 32. Il peut s'agir là encore de voiles de carde. Les couches d'éléments gommants, 34 et 34' respectivement, sont prises en sandwich entre les couches 32 et 36 pour l'une et les couches 32 et 36' pour l'autre.

Sur la figure 4, on a représenté une réalisation où les éléments gommants 44 sont répartis sur la surface de la première couche 42 de manière régulièrement espacée. Par exemple les éléments peuvent être disposés en lignes ou en bandes ou selon tout autre motif continu ou discontinu. On obtient ainsi un relief bien marqué par alternance de bosses et de surfaces planes. Comme dans les autres réalisations, ils sont maintenus par la couche fibreuse supérieure 46 plus légère que la couche 42.

Selon une autre structure non représentée, on dispose un ou plusieurs voiles de carde sur la face libre de la première couche, de façon à rendre le contact de la face opposée à la face gommante plus doux.

L'invention ne se limite pas aux modes de réalisation présentés ci-dessus, elle englobe toutes les variantes à la portée de l'homme du métier, c'est-à-dire qui peuvent se déduire de façon claire et facile des revendications.

## Revendications

1. Tampon pour le soin de la peau comprenant des éléments gommants (4, 24, 34-34', 44), **caractérisé en ce que** lesdits éléments gommants sont répartis entre au moins une première couche fibreuse (2, 22, 32, 42) et une seconde couche fibreuse (6, 26, 36-36', 46) de plus faible grammage que la première couche fibreuse.

2. Tampon selon la revendication précédente dont les éléments gommants forment une couche.

3. Tampon selon la revendication précédente dont la couche d'éléments gommants est uniformément répartie.

4. Tampon selon la revendication 2 dont la couche d'éléments gommants est répartie selon un motif continu ou discontinu.

5. Tampon selon la revendication 1 dont les couches fibreuses (2, 22, 32, 42, 6, 26, 36-36', 46) sont composées de fibres choisies parmi les fibres cellulosiques, artificielles ou synthétiques, seules ou en mélange.

6. Tampon selon la revendication précédente dont la première couche (2, 22, 32, 42) est composée de fibres choisies parmi les fibres de coton, viscose, polyester ou polypropylène seules ou en mélange.

7. Tampon selon la revendication précédente dont le grammage de la première couche (2, 22, 32, 42) est compris entre 20 et 350 g/m².

8. Tampon selon la revendication précédente dont la première couche (2, 22, 32, 42) est une nappe de fibres, formée sur une machine de type RANDO WEBBER.

9. Tampon selon la revendication 5 dont la seconde couche (6, 26, 36-36', 46) est composée de fibres choisies parmi les fibres de coton, lin, ramie, viscose, polyester ou polypropylène, seules ou en mélange.

10. Tampon selon la revendication précédente, dont le grammage de la seconde couche (6, 26, 36-36', 46) est compris entre 5 et 50 g/m².

11. Tampon selon la revendication précédente dont la seconde couche (6, 26, 36-36', 46) est composée d'un ou plusieurs voiles de carde.

12. Tampon selon l'une des revendications précédentes dont le grammage de la couche d'éléments gommants (4, 24, 34-34', 44) est compris entre 2 et 50 g/m².

13. Tampon selon la revendication précédente, dont les éléments gommants (4, 24, 34-34', 44) sont choisis parmi les produits,
- organiques naturels tels que : akènes de fraise, noyaux d'abricot, silice organique de bambou ou cellulose de courge,
- minéraux tels que les billes de silice,
- artificiels tels que des sphères de cellulose et de méthylcellulose
- ou bien encore synthétiques tels que les polymères polyéthylène, nylon, polypropylène ou EVA.

14. Tampon selon l'une des revendications précédentes comprenant une deuxième couche d'éléments gommants (34') compris entre la première couche (32) et une troisième couche fibreuse (36').

15. Tampon selon l'une des revendications précédentes dont les couches fibreuses sont liées.

16. Tampon selon l'une des revendications précédentes dont les couches sont liées entre elles.

17. Tampon selon l'une des revendications 15 ou 16 dont les fibres sont liées entre elles par liage hydraulique ou mécanique.

18. Tampon selon la revendication 15 ou 16 comprenant au moins de la matière thermofusible et dont les fibres sont au moins partiellement thermoliées.

19. Procédé de fabrication d'un tampon selon l'une des revendications précédentes comprenant les étapes suivantes : formation d'une première couche de fibres, dépôt d'une couche d'éléments gommants sur la couche, **caractérisé en ce qu'**il consiste en outre au dépôt d'une deuxième couche de fibres sur la couche d'éléments gommants, la deuxième couche de fibres étant de plus faible gommage que la première couche de fibres.

## Claims

1. Pad for skin care comprising exfoliating elements (4, 24, 34-34', 44) **characterized in that** said exfoliating elements are distributed between at least one first fibrous layer (2, 22, 32, 42) and a second fibrous layer (6, 26, 36-36', 46) of lower basis weight than the first fibrous layer.

2. Pad according to the preceding claim, wherein the exfoliating elements form a layer.

3. Pad according to the preceding claim, wherein the layer of exfoliating elements is evenly distributed.

4. Pad according to Claim 2, wherein the layer of exfoliating elements is distributed in a continuous or broken pattern.

5. Pad according to Claim 1, wherein the fibrous layers (2, 22, 32, 42, 6, 26, 36-36', 46) consist of fibres selected from among cellulose fibres, artificial fibres, or synthetic fibres, individually or in a mixture.

6. Pad according to the preceding claim, wherein the first layer (2, 22, 32, 42) consists of fibres selected from among the fibres of cotton, viscose, polyester, or polypropylene, individually or in a mixture.

7. Pad according to the preceding claim, wherein the basis weight of the first layer (2, 22, 32, 42) ranges from 20 to 350 g/m².

8. Pad according to the preceding claim, wherein the first layer (2, 22, 32, 42) is a sheet of fibres formed on a machine of the Rando Webber type.

9. Pad according to Claim 5, wherein the second layer (6, 26, 36-36', 46) consists of fibres selected from among the fibres of cotton, flax, ramie, viscose, polyester, or polypropylene, individually or in a mixture.

10. Pad according to the preceding claim, wherein the basis weight of the second layer (6, 26, 36-36', 46) ranges from 5 to 50 g/m².

11. Pad according to the preceding claim, wherein the second layer (6, 26, 36-36', 46) consists of one or more carded webs.

12. Pad according to one of the preceding claims, wherein the basis weight of the layer of exfoliating elements (4, 24, 34-34', 44) ranges from 2 to 50 g/m².

13. Pad according to the preceding claim, wherein the exfoliating elements (4, 24, 34-34', 44) are selected from among
- natural organic products, such as strawberry achenes, apricot kernels, organic bamboo silica, or gourd cellulose,
- mineral products, such as beads of silica,
- artificial products, such as spheres of cellulose and methylcellulose,
- or synthetic products, such as the polymers polyethylene, nylon, polypropylene, or EVA.

14. Pad according to one of the preceding claims comprising a second layer of exfoliating elements (34') introduced between the first layer (32) and a third fibrous layer (36').

15. Pad according to one of the preceding claims, wherein the fibrous layers are bonded.

16. Pad according to one of the preceding claims, wherein the layers are bonded to each other.

17. Pad according to either of Claims 15 and 16, wherein the fibres are bonded to each other by hydraulic bonding or mechanical bonding.

18. Pad according to Claim 15 or 16, comprising at least thermofusible material and the fibres of which pad are at least in part thermally bonded.

19. Process of manufacturing a pad according to one of the preceding claims, comprising the following stages: formation of a first layer of fibres, depositing of a layer of exfoliating elements on the layer, **characterized in that** it furthermore consists in depositing a second layer of fibres on the layer of exfoliating elements, the second layer of fibres being of lower basis weight than the first layer of fibres.

## Patentansprüche

1. Hautpflege-Pad, das Peeling-Elemente (4, 24, 34-34', 44) enthält, **dadurch gekennzeichnet, dass** die Peeling-Elemente zwischen mindestens einer ersten Faserschicht (2, 22, 32, 42) und einer zweiten Faserschicht (6, 26, 36-36', 46) geringeren Flächengewichts als die erste Faserschicht verteilt sind.

2. Pad nach dem vorhergehenden Anspruch, dessen Peeling-Elemente eine Schicht formen.

3. Pad nach dem vorhergehenden Anspruch, dessen Schicht von Peeling-Elementen gleichmäßig verteilt ist.

4. Pad nach Anspruch 2, dessen Schicht von Peeling-Elementen gemäß einem durchgehenden oder unterbrochenen Muster verteilt ist.

5. Pad nach Anspruch 1, dessen Faserschichten (2, 22, 32, 42, 6, 26, 36-36', 46) aus Fasern zusammengesetzt sind, die aus den künstlichen oder synthetischen Cellulosefasern, einzeln oder gemischt, ausgewählt sind.

6. Pad nach dem vorhergehenden Anspruch, dessen erste Schicht (2, 22, 32, 42) aus Fasern zusammengesetzt ist, die aus den Baumwoll-, Viskose-, Polyester- oder Polypropylenfasern, alleine oder gemischt, ausgewählt sind.

7. Pad nach dem vorhergehenden Anspruch, dessen Flächengewicht der ersten Schicht (2, 22, 32, 42) zwischen 20 und 350 g/m² beträgt.

8. Pad nach dem vorhergehenden Anspruch, dessen erste Schicht (2, 22, 32, 42) eine Lage von Fasern ist, die auf einer Maschine des Typs RANDO WEBBER geformt ist.

9. Pad nach Anspruch 5, dessen zweite Schicht (6, 26, 36-36', 46) aus Fasern zusammengesetzt ist, die aus den Baumwoll-, Leinen-, Ramie-, Viskose-, Polyester- oder Polypropylenfasern, einzeln oder gemischt, ausgewählt sind.

10. Pad nach dem vorhergehenden Anspruch, dessen Flächengewicht der zweiten Schicht (6, 26, 36-36', 46) zwischen 5 und 50 g/m² beträgt.

11. Pad nach dem vorhergehenden Anspruch, dessen zweite Schicht (6, 26, 36-36', 46) aus einem oder mehreren Kardenbändern zusammengesetzt ist.

12. Pad nach einem der vorhergehenden Ansprüche, dessen Flächengewicht der Schicht von Peeling-Elementen (4, 24, 34-34', 44) zwischen 2 und 50 g/m² liegt.

13. Pad nach dem vorhergehenden Anspruch, dessen Peeling-Elemente (4, 24, 34-34', 44) unter den folgenden Produkten ausgewählt sind
- organische natürliche, wie: Erdbeer-Achänen, Aprikosenkerne, organische Bambus-Kieselsäure oder Kürbis-Cellulose,
- mineralische wie Kieselsäurekügelchen,
- künstliche wie Cellulose- und Methylcellulosekugeln,
- oder auch synthetische wie Polyethylen-, Nylon-, Polypropylen-Polymere oder EVA.

14. Pad nach einem der vorhergehenden Ansprüche, das eine zweite Schicht von Peeling-Elementen (34') enthält, die zwischen der ersten Schicht (32) und einer dritten Faserschicht (36') liegt.

15. Pad nach einem der vorhergehenden Ansprüche, dessen Faserschichten verbunden sind.

16. Pad nach einem der vorhergehenden Ansprüche, dessen Schichten miteinander verbunden sind.

17. Pad nach einem der Ansprüche 15 oder 16, dessen Fasern durch hydraulische oder mechanische Bindung miteinander verbunden sind.

18. Pad nach dem Anspruch 15 oder 16, das mindestens einen wärmeschmelzbaren Stoff enthält und dessen Fasern zumindest teilweise wärmeverbunden sind.

19. Verfahren zur Herstellung eines Pads nach einem der vorhergehenden Ansprüche, das die folgenden Schritte enthält: Formen einer ersten Schicht von Fasern, Aufbringen einer Schicht von Peeling-Elementen auf die Schicht, **dadurch gekennzeichnet, dass** es außerdem aus dem Aufbringen einer zweiten Schicht von Fasern auf die Schicht von Peeling-Elementen besteht, wobei die zweite Schicht von Fasern ein geringeres Flächengewicht hat als die erste Schicht von Fasern.
